# EUROPÄISCHE PATENTSCHRIFT

(11) **EP 0 395 778 B1**
(45) Veröffentlichungstag und Bekanntmachung des Hinweises auf die Patenterteilung: **06.10.1993**
(21) Anmeldenummer: 89108034.3
(22) Anmeldetag: 03.05.1989
(51) Int. Cl.: A61M 39/04

(54) **Vorrichtung mit implantierbarer Infusionskammer und einem sich von dieser forterstreckenden Katheter**
Device with an implantable reservoir and catheter extending therefrom
Dispositif avec réservoir implantable et un cathéter s'étendant à partir de celui-ci

(43) Veröffentlichungstag der Anmeldung: 07.11.1990
(73) Patentinhaber: Strecker, Ernst Peter, Dr.-med.Prof., D-76228 Karlsruhe (DE)
(72) Erfinder: Strecker, Ernst Peter, Dr.-med.Prof., D-76228 Karlsruhe (DE)
(74) Vertreter: Geitz, Heinrich, Dr.-Ing.

(56) Entgegenhaltungen:
- EP-A- 0 119 596
- EP-A- 0 233 986
- EP-A- 0 260 080
- EP-A- 0 268 108
- WO-A-88/04914
- DE-A- 3 837 779
- US-A- 3 971 376

## Beschreibung

Die Erfindung bezieht sich auf eine Vorrichtung mit einer in das Unterhaut-Fettgewebe eines Patienten implantierbaren Infusionskammer, die einen mittels einer Injektionsnadel durchstechbaren Bereich aus elastischem Material besitzt, und mit einem sich von der Infusionskammer forterstreckenden und in ein Blutgefäß, wie eine Arterie, oder eine andere Körperhöhle einführbaren Katheter.

Aus der DE-PS 31 02 993 ist bereits eine subkutan implantierbare Vorrichtung zum Zuführen flüssiger Arzneimittel bekannt, die eine Infusionskammer mit einer durchstechbaren Membran als Punktionsfenster sowie einen an die Kammer angeschlossenen Schlauch zur Medikamentenweiterleitung besitzt. Dem Punktionsfenster gegenüberliegend ist eine Perforationen der Kammerrückwand verhindernde Verstärkung angeordnet.

In der DE-PS 33 09 788 ist eine ähnliche Vorrichtung mit einer ein durchstechbares Punktionsfenster aufweisenden Infusionskammer und einem mit dieser verbindbaren Katheter beschrieben. Die als "Zuspritzteil" bezeichnete Infusionskammer besitzt einen das Punktionsfenster umschließenden und über dieses vorstehenden Randwulst sowie letzterem gegenüberliegend an der Unterseite eine peripher über die Kammer vorstehende Platte mit über den Umfang verteilt angeordneten Lochungen zur Fixation mittels Fäden im implantierten Zustand.

Aus der EP-A-0 260 080 ist eine Vorrichtung ganz ähnlicher Zweckbestimmung bekannt, die eine subkutan implantierbare Infusionskammer mit einer Kanülenstop-Platte und einem mittels einer Kanüle durchstechbaren Bereich in der der Kanülenstop-Platte gegenüberliegenden Kammerwand besitzt. Bei dieser Vorrichtung ist die Infusionskammer an einem Ende einer Trag- und Einführschiene aufgenommen, die in ihrer Gesamtheit durch einen Hautschnitt beträchtlicher Größe chirurgisch operativ implantiert werden muß. Ferner ist auch Unbefriedigend, daß der durchstechbare Bereich in der Kammerwand zumindest auf Dauer keinen hinreichend sicheren Verschluß eines Stichkanals nach dem Punktieren gewährleistet.

Vorrichtungen dieser Art ermöglichen einen dauerhaften venösen oder arteriellen Gefäßzugang. Für arterielle Gefäßzugänge bestimmte Vorrichtungen unterscheiden sich von venösen Systemen durch ein Ventil an der Katheterspitze, um einer Okklusion durch zurückströmendes Blut im arteriellen Hochdrucksystem durch Thrombosenbildung vorzubeugen.

Derartige Vorrichtungen, die zum selektiven Einschleußen von Medikamenten in Gefäße dienen, sind subkutan implantierbar, was einen chirurgischen Eingriff jedenfalls in Lokalanästhesie voraussetzt.

Bei einer bekannten Implantationsmethode wird nach dem Legen eines Hautschnittes das Gefäß, in das ein Katheter eingeführt werden soll, beispielsweise mittels einer Punktionskanüle oder auf andere geeignete Weise eröffnet und dann der Katheter in das Gefäß eingeführt. Danach ist eine epifasciale Tasche für die Infusionskammer zu präparieren und letztere so in der Tasche zu plazieren, daß ein als Membran ausgebildetes Punktionsfenster außerhalb der Schnittstelle unter der Haut liegt. Nach dem Implantieren und nach dem gegebenenfalls vorherigen Legen einer Saugdrainage ist die Wunde mit Subkutan- oder Hautnähten zu verschließen.

Wenn aus anatomischen Gründen der Gefäßzugang nur weiter entfernt von der vorgesehenen Position der Infusionskammer möglich ist, muß im Bereich dieses Zuganges ein Hautschnitt gelegt und der Katheter von einer subkutanen Tasche zum Aufnehmen der Infusionskammer durch einen subkutanen Tunnel zu dem Hautschnitt verlegt werden, bevor der Katheter in das Gefäß eingeführt werden kann.

Die subkutan implantierbaren Vorrichtungen sind insbesondere zur intraarteriellen Chemotherapie geeignet, die eine regionale Tumortherapie von Primärtumoren und von Metastasen erlaubt. Zur Planung vor Implantation muß eine Arteriographie durchgeführt werden, um exakte Kenntnis über die anatomische Gefäßversorgung eines tumortragenden Organs zu erhalten und somit den Ort der Katheterspitze im Gefäßsystem zur Medikamentenabgabe zu bestimmen.

Der Vorteil der intraarteriellen Methode besteht darin, daß höhere Konzentrationen der einzusetzenden Chemotherapeutika in das Tumorgewebe gebracht werden können, die somit eine gezielte Behandlung mit geringeren allgemeinen Nebenwirkungen erlauben. Das intraarteriell abzugebende Medikament läßt sich nur durch Punktion der Haut und der Membran des Punktionsfensters in die Infusionskammer und von dort selektiv in die tumorversorgende Arterie über das in letztere eingeführte Schlauchsystem abgeben.

Unbefriedigend bei den bekannten Kathetersystemen indessen ist, daß die Infusionskammern intraoperativ chirurgisch imlantiert und bei vom Gefäßzugang entfernter Positionierung der Infusionskammer ein weiterer Hautschnitt gelegt sowie der Katheter durch einen subkutanen Tunnel zu dem Hautschnitt verlegt werden müssen, bevor eine Kathetereinführung in das Gefäß vorgenommen werden kann. Es ist somit immer eine größere Operation notwendig, wie z.B. bei Lebertumoren eine Laparotomie, um den Katheter exakt einführen und die Infusionskammer plazieren zu können. Die mit einer derartigen Operation verbundene Belastung ist für Tumorpatienten häufig nicht zumutbar, da beispielsweise bei Lebermetastasen eines primären Colon-Carcinoms eine durchschnittliche Lebenserwartung von nur noch etwa sechs Monaten besteht.

Ziel der Erfindung ist die Schaffung einer Vorrichtung der vorgenannten Zweckbestimmung, die weniger invasiv als die subkutan implantierbaren Systeme ist und demgemäß einfacher und Patienten weniger belastend implantiert werden kann, bei der ferner aber auch eine dauerhaft wirksame Abdichtung der Stichkanäle nach dem Punktieren zwecks Nachbefüllung sichergestellt ist.

Gelöst ist diese Aufgabe durch die Ausbildung der Vorrichtung nach dem Oberbegriff des Patentanspruchs 1 als perkutan durch eine Hautpunktionsöffnung implantierbares System, dessen Infusionskammer aus einem langgestreckten formfesten Hohlkörper mit einem Punktionsfenster aus unter Vorspannung stehendem durchstechbarem und die Einstichstelle selbsttätig schließendem Material besteht.

Bei der erfindungsgemäßen Vorrichtung, die im Aufbau äußerst einfach ist, handelt es sich somit um ein Infusionskammersystem mit einer langgestreckten schlanken Infusionskammer, die nicht intraoperativ chirurgisch unter die Haut im Unterhaut-Fettgewebe eingesetzt werden muß, sondern die Implantation erfolgt perkutan durch eine vorher mittels eines Dilators aufgeweitete Punktionsöffnung der Haut hindurch. Dies ist weitaus weniger invasiv als eine intraoperative Implantation, mithin also patientenschonend.

Ein weiterer wichtiger Vorteil der erfindungsgemäßen Vorrichtung besteht darin, daß auch bei häufigem Nachfüllen der Infusionskammer mittels einer das Punktionsfenster durchstechenden Kanüle ein dauerhaft sicherer Wiederverschluß der Einstichkanäle gewährleistet ist. Ursächlich dafür ist, daß das Material des Punktionsfensters unter Vorspannung steht.

Gemäß einer Weiterbildung der Erfindung kann es sich bei dem Punktionsfenster um einen die Infusionskammer nach außen abschließenden Stopfen aus Silikon oder einem ähnlichen Material handeln, der unter Vorspannung in einem Teilbereich der Infusionskammer aufgenommen ist. Zweckmäßigerweise kann das Punktionsfenster im Bereich einer die Kammerwand durchbrechenden Ausnehmung angeordnet sein, die nach Implantation unter der Haut des Patienten liegt und mithin ein im wesentlichen "senkrechtes" Punktieren ermöglicht.

Als gleichfalls vorteilhaft hat sich erwiesen, wenn in die Außenzone des das Punktionsfenster bildenden Materials ein das Ausweichen des unter Vorspannung stehenden Fenstermaterials verhinderndes Gitter integriert ist, das aus geeignetem festem Material besteht.

Ebenfalls gemäß einer Weiterbildung kann sich um das Punktionsfenster ein über dieses kammeraußenseitig vorstehender Randwulst herumerstrecken, der durch die Haut des Patienten ertastbar ist und mithin das Punktieren der Infusionskammer erleichtert.

Eine nochmals andere Weiterbildung sieht vor, daß die Infusionskammer und der Katheter mittels einer Steckverbindung trennbar miteinander verbunden sind. Diese Ausgestaltung ermöglicht ein besonders einfaches Implantieren in der Weise, daß zunächst der Katheter über einen zuvor eingebrachten Führungsdraht in beispielsweise ein zu punktierendes Gefäß eingeführt und dann mit der Infusionskammer verbunden wird, die zuvor oder auch danach in eine von der Punktionsstelle aus in das Unterhaut-Fettgewebe eines Patienten eingebrachte Tunnelung implantiert worden ist.

Eine besonders vorteilhafte Lagestabilisierung der Infusionskammer nach deren Implantation ist nach einem weiteren Merkmal der Erfindung dann erzielbar, wenn die Infusionskammer mit sich außenseitig von der Kammerwand forterstreckenden federnden Verankerungsmitteln versehen ist, die auf der - im implantierten Zustand - Unterseite und gegebenenfalls auf den Stirnwänden der Infusionskammer angeordnet sind und in das die Kammer umgebende Unterhaut-Fettgewebe eingreifen. Derartige Verankerungsmittel können gleichermaßen bei im Querschnitt veränderbarer und formstabiler Infusionskammer vorgesehen sein.

Bei den Verankerungsmitteln kann es sich um in der Gebrauchslage von der Kammerwand der Infusionskammer außenseitig vorstehende Ankerstifte oder auch um Schlingen handeln, deren Enden jeweils mit der Kammerwand verbunden sind. Bei derartigen Verankerungsmitteln hat sich als vorteilhaft erwiesen, wenn als Implantationshilfe die Infusionskammer in einer nach der Implantation abstreifbaren äußeren Hülle aufgenommen ist und mittels dieser die Verankerungsmittel an die Kammerwand angelegt sind. Nach dem Abziehen der äußeren Hülle spreizen sich dann die Verankerungsmittel auf und vermitteln eine präzise Lagestabilisierung der Infusionskammer, so daß diese bei Punktion weder seitlich ausweichen noch sich in Längsrichtung verschieben kann.

Eine das perkutane Einführen erleichternde andere Weiterbildung der Erfindung sieht vor, daß die Infusionskammer mit einem sich in Einführrichtung erstreckenden und zur Infusionskammer hin erweiternden Einführkonus vorgesehen ist, der das Aufdehnen der Hautöffnung beim Einführen der Infusionskammer begünstigt.

Zweckmäßigerweise kann der Einführkonus eine flache Form aufweisen und bei solchen Vorrichtungen, bei denen sich der Katheter auf der Seite des Einführkonus forterstreckt, im implantierten Zustand durch das Unterhaut-Fettgewebe bis beispielsweise an eine Arterie heranreichen, so daß eine Abstützung der Infusionskammer gegenüber dem punktierten Gefäß gewährleistet ist und eine zusätzliche Abdichtung des Punktionsloches der Gefäßwand erzielt wird.

Schließlich kann, ebenfalls gemäß einer die Implantation erleichternden Weiterbildung, die Infusionskammer auch in einer nach der Implantation entgegen der Einführrichtung abziehbaren Einführhülse aufgenommen sein.

Anhand der beigefügten Zeichnung sollen nachstehend einige Ausführungsbeispiele der erfindungsgemäßen Vorrichtung sowie deren bestimmungsgemäße Verwendung erläutert werden. In schematischen Ansichten zeigen:
- Fig. 1: in einer teilweisen Längsschnittansicht ein Kathetersystem mit einer formfesten Infusionskammer im implantierten Zustand
- Fig. 2: eine gegenüber Fig. 1 abgewandelte Ausführungsform mit einem im Bereich eines Endes der Infusionskammer eingesetzten Verschlußstopfens die Kammerwand durchbrechenden Punktionsfenster und mit kammerunterseitig angeordneten Verankerungsmitteln,
- Fig. 3: einen Querschnitt gemäß der Schnittlinie III-III in Fig. 2 durch die Infusionskammer mit freigegebenen Verankerungsmitteln,
- Fig. 4: in einer Ansicht ähnlich Fig. 2 eine Abwandlungsform des Kathetersystems mit formfester Infusionskammer,
- Fig. 5: in einer die Schnittlinie V-V in Fig. 4 entsprechenden Schnittansicht eine weitere Abwandlungsform der Infusionskammer,
- Fig. 6: in einer Schnittansicht wie in Fig. 5 eine zu letzterer alternive Querschnittsausbildung der Infusionskammer,
- Fig. 7: die in Fig. 4 veranschaulichte Vorrichtung in verkleinerter Darstellung bei bestimmungsgemäßer Verwendung mit einem bereits in ein Blutgefäß eingeführten Katheter und mit der Infusionskammer während des Einführens in eine sich von einer Hautpunktionsstelle forterstreckende Tunnelung und
- Fig. 8: einen Querschnitt gemäß der Schnittlinie VIII-VIII in Fig. 7 durch die Infusionskammer und eine - in die Tunnelung im Unterhaut-Fettgewebe eines Patienten eingeführte - geschlitzte Einführhülse.

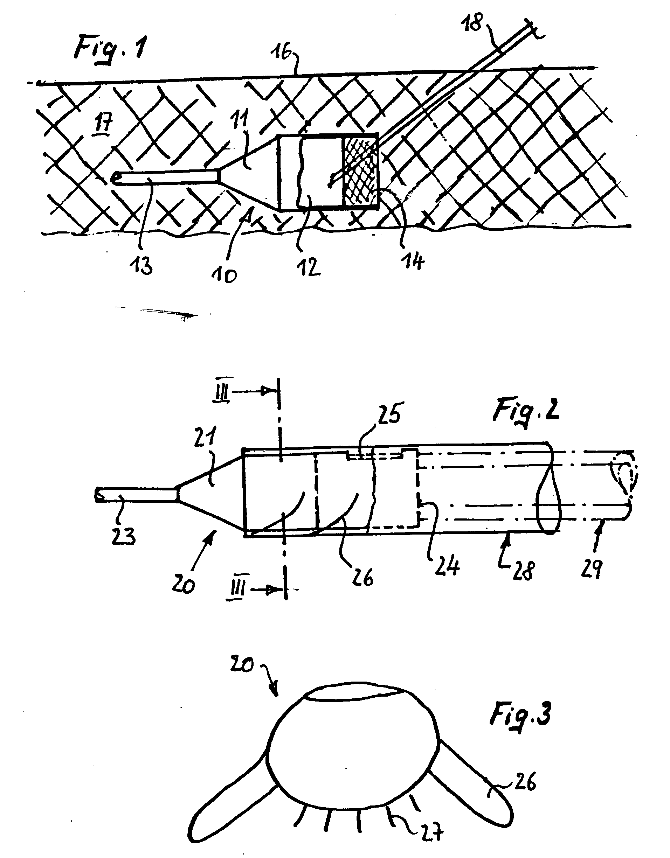

Das in Fig. 1 veranschaulichte Kathetersystem besitzt eine als länglicher formstabiler Hohlkörper ausgebildete Infusionskammer 10 aus gewebeverträglichem Kunststoff mit einem Einführkonus 11 an einem Ende. Von dem Einführkonus erstreckt sich ein in ein Gefäß einführbarer und mit dem Innenraum 12 der Infusionskammer in Verbindung stehender Katheter 13 fort. Am anderen Ende ist die Infusionskammer mittels eines in die Kammer eingesetzten Verschlußstopfens 14 in Form einer starken Silikonplatte abgeschlossen.

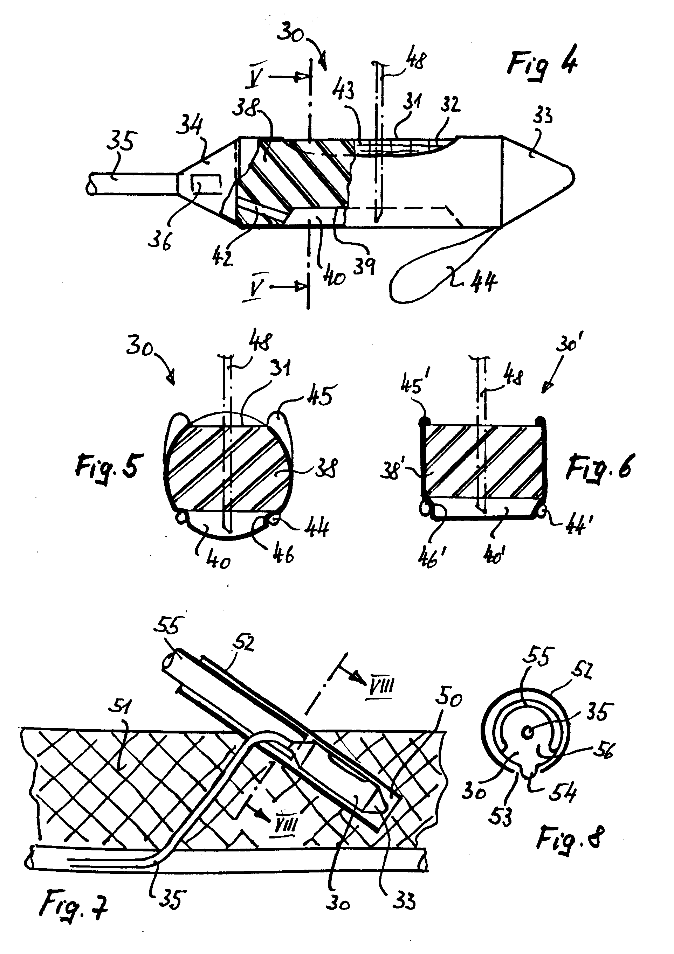

Das in den Fig. 2 und 3 veranschaulichte Kathetersystem besitzt eine Infusionskammer 20 aus gewebeverträglichem Kunststoff, die ebenfalls als länglicher formstabiler Hohlkörper ausgebildet und an ihrem einen Ende von einem Einführkonus 21 abgeschlossen ist. Von diesem Einführkonus erstreckt sich wieder ein in ein Gefäß einführbarer und mit dem Innenraum der Infusionskammer 20 in Verbindung stehender Katheter 23 fort. Das andere Ende der Infusionskammer ist mittels eines in diese eingesetzten Verschlußstopfens 24 verschlossen. Im Unterschied zu der Ausführungsform nach Fig. 1 ist die Kammerwand der Infusionskammer mit einer Ausnehmung 25 als Punktionsfenster versehen und der Verschlußstopfen 24 reicht über dieses Punktionsfenster in Richtung zum Einführkonus 21 hinaus.

Ferner unterscheidet sich die Infusionskammer 20 des in den Fig. 2 und 3 veranschaulichten Kathetersystems von der Infusionskammer 10 gemäß Fig. 1 auch dadurch, daß die Außenwand kammerunterseitig mit umlegbaren Verankerungsschlingen 26 und/oder mit umlegbaren Verankerungsstiften 27 versehen ist und daß diese Verankerungsmittel durch eine die Infusionskammer umschließende Einführhülse 28 nach vorn gerichtet, also in Einführrichtung, an die Kammerwand angedrückt sind. Die Einführhülse ist nach der Implantation nach der mittels des Verschlußstopfens 24 verschlossenen Seite der Infusionskammer hin abziehbar.

Implantiert werden die Systeme nach den Fig. 1 bis 3, indem in hier nicht weiter interessierender Weise zunächst ein Führungsdraht durch eine Hautpunktionsöffnung bis in beispielsweise eine Arterie eingebracht und dann der Katheter mit der Infusionskammer über den Führungsdraht aufgeschoben wird, wobei sich der Führungsdraht durch den Katheter, die Infusionskammer und die diese auf der vom Katheter entfernten Seite abschließenden Verschlußstopfen hindurcherstreckt. Nach dem lagerichtigen Plazieren wird der Führungsdraht in bekannter Weise zurückgezogen, wobei sich die Durchgangsöffnung in dem die Infusionskammer rückseitig abschließenden Verschlußstopfen aus Silikon oder einem anderen geeigneten Material selbsttätig schließt.

In Fig. 1 ist das Kathetersystem im implantierten Zustand unter der Haut 16 im Unterhaut-Fettgewebe 17 eingebettet gezeigt. Eine Punktion der Infusionskammer, beispielsweise zwecks Medikamentenzufuhr, gelingt in einfacher Weise, indem mittels einer Injektionsnadel 18 die Haut 16 und das Unterhaut-Fettgewebe 17 sowie der die Infusionskammer rückseitig abschließende Verschlußstopfen 14 durchstochen werden. Angesichts der Vorspannung des Verschlußstopfenmaterials schließt sich die Einstichstelle des Verschlußstopfens 24 nach dem Zurückziehen der Injektionsnadel wieder selbsttätig. Dies ist in Fig. 1 schematisch angedeutet.

Nach der Implantation des Kathetersystems nach den Fig. 2 und 3 wird die Einführhülse 28 nach der vom Einführkonus 21 entfernten Seite von der Infusionskammer 20 abgezogen, worauf sich die während des Implantierens an die Kammerwand angelegten Verankerungsmittel 26, 27 in der aus Fig. 3 ersichtlichen Weise aufrichten und in das der Kammerunterseite benachbarte Unterhaut-Fettgewebe des Patienten eindringen. Dadurch wird eine durchaus erwünschte Lagestabilisierung der Infusionskammer verwirklicht. Während des Abziehens der Einführhülse 28 von der Infusionskammer 20 muß letztere mittels eines in Fig. 2 strichpunktiert angedeuteten Gegenhalters 29 in ihrer bestimmungsgemäßen Lage gehalten werden.

Bei der in Fig. 4 dargestellten Ausführungsform ist die Infusionskammer 30 ebenfalls formfest und als langgestrecktes Hülsenteil mit einer den Hülsenmantel zwecks Bildung eines Punktionsfensters 31 durchbrechenden Ausnehmung 32 ausgebildet. An dem einen Ende ist der Hülsenmantel von einem angeformten Einführkonus 33 abgeschlossen, während sich von einem ebenfalls annähernd konisch ausgebildeten Abschnitt 34 am anderen Ende ein Katheter 35 forterstreckt, der fest oder auch trennbar über eine nicht gezeigte Steckverbindung angeschlossen ist. Das katheterseitige Endteil des Hülsenmantels ist ferner mit einer den sicheren Angriff einer Pinzette oder einer Klemme ermöglichenden Einmuldung 36 versehen.

Bei dieser Ausbildung ist innerhalb des Hülsenmantels ein sich zwischen den beiden endseitigen Konen 33, 34 erstreckender Silikonpfropfen 38 unter radialer Vorspannung aufgenommen, der im Bereich seiner beiden stirnseitigen Enden den Hülsenmantel voll ausfüllt, jedoch auf der dem Punktionsfenster 31 bzw. der dieses bildenden Ausnehmung 32 im Hülsenmantel gegenüberliegenden Seite bei 39 abgeflacht ist, so daß sich dadurch ein durch den Silikonpfropfen 38 hindurch punktierbarer Innenraum 40 für die Aufnahme einer Infusionslösung oder eines Medikaments bildet. Von diesem Aufnahmeraum erstreckt sich ein in dem katheterseitigen Endteil 34 des Hülsenmantels ausmündender Kanal 42 durch den Silikonpfropfen 38 hindurch. Zumindest im Bereich der das Punktionsfenster bildenden Ausnehmung ist der Silikonpfropfen 38 mit einem in seine Oberfläche integrierten Gitter 43 aus formfesten Fadenmaterial versehen, das ein radiales Ausweichen des Silikonpfropfens im Bereich der genannten Ausnehmung verhindert. Schließlich sind in der oben bereits in Verbindung mit den Fig. 2 und 3 erläuterten Weise auf der vom Katheter 35 abgewandten Seite außenseitig am Hülsenmantel Anker 44 in Form von im implantierten Zustand sich abspreizenden Schlingen angeordnet, die nach der Implantation eine sichere Festlegung der Infusionskammer 30 im Unterhaut-Fettgewebe eines Patienten vermitteln.

Bei der in Fig. 5 in einem Querschnitt gemäß der Schnittlinie V-V in Fig. 4 veranschaulichten Ausführungsform hat der die Infusionskammer 30 bildende Hülsenmantel eine etwa ovale Form und im Bereich des das Punktionsfenster bildenden Ausnehmung 32 des Hülsenmantels erstrecken sich beidseitig dieser Ausnehmung über das Punktionsfenster 31 vorstehende Wülste 45 entlang, die im implantierten Zustand durch die Haut des Patienten hindurch ein Ertasten der Lage des Punktionsfensters ermöglichen. Auf der vom Punktionsfenster abgewandten Seite, die im implantierten Zustand, bezogen auf die Haut eines Patienten, die Unterseite der Infusionskammer bildet, sind längslaufende Einformungen 46 im Hülsenmantel vorgesehen, in denen die beim Implantieren angelegten Ankerschlingen 44 aufgenommen sind und die gleichzeitig eine unterseitige Abstützung für den innerhalb des Hülsenmantels aufgenommenen Silikonpfropfen 38 bilden.

Beim Punktieren der Infusionskammer wird durch das Punktionsfenster 31 hindurch der Silikonpfropfen 38 mittels einer Kanüle 48 durchstochen, die in den Fig. 4 und 5 angedeutet ist. Nach dem Zurückziehen der Kanüle schließt sich angesichts der Vorspannung des Silikonmaterials die Einstichstelle selbsttätig, so daß jederzeit eine Nachfüllung des Medikamentendepots möglich ist, aber in dem Aufnahmeraum 40 deponierte Medikamente nur über den Katheter abströmen können.

Fig. 6 zeigt in einer Ansicht ähnlich Fig. 5 eine alternative Ausbildung mit einer im wesentlichen Rechteckquerschnitt aufweisenden Infusionskammer 30', die im Bereich ihres von dem Katheter entfernten Endes gleichfalls mit nur angedeuteten Verankerungsmitteln 44' versehen ist. Auch bei dieser Ausführungsform erstrecken sich beidseitig des Infusionsfensters 31' über dieses hinausragende Wülste 45', die durch die Haut eines Patienten hindurch ein Ertasten der Lage des Infusionsfensters ermöglichen.

Fig. 7 schließlich zeigt in gegenüber den Fig. 4 bis 6 stark verkleinerter Darstellung die Implantation einer derartigen Infusionskammer 30, 30', deren eines Ende mit einem bereits in ein Gefäß eingeführten Katheter 35 verbunden ist. Die Infusionskammer wird dabei in eine von einer Hautpunktionsstelle aus mittels eines - nicht gezeigten - Dilators erweiterte Kammer 50 im Unterhaut-Fettgewebe 51 eines Patienten mit dem vom Katheter abgewandten Ende eingeführt, wobei dieses Einführen durch den Einführkonus 33 an dem vom Katheter abgewandten Ende der Infusionskammer erleichtert wird. Als Einführhilfe wird dabei durch die erweiterte Hauptpunktionsöffnung hindurch eine geschlitzte Einführhülse 52 in die Kammer 50 eingeschoben und dann die Infusionskammer durch die Einführhülse hindurch eingeführt, wobei der mit der Infusionskammer verbundene Katheter sich durch einen Längsschlitz 53 der Einführhülse hindurcherstreckt.

Zweckmäßigerweise kann auch, wie Fig. 8 andeutet, die Infusionskammer 30 auf der vom Punktionsfenster 31 abgewandten Seite mit einer Längsrippe 54 versehen sein, die dann in den Längsschlitz 53 der Einführhülse 52 eingreift und dadurch eine lagerichtige Plazierung der Infusionskammer vermittelt. Als weitere Einführhilfe dient ein Pusher 55, der korrespondierend zu dem Längsschlitz 53 der Einführhülse 52 mit einem Längsschlitz 56 versehen ist und mittels dessen die mit dem Katheter verbundene Infusionskammer durch die Einführhülse 52 hindurch in die vorbereitete Aufnahmekammer 50 einschiebbar ist. Nach dem lagerichtigen Plazieren der Infusionskammer werden aufeinanderfolgend zunächst die Einführhülse 52 und dann der Pusher 55 zurückgezogen, worauf es lediglich noch des Verschließens der Hautpunktionsöffnung bedarf, um die Implantation zu beenden.

Auch bei den anhand der Fig. 4 bis 8 erläuterten Ausführungsformen gelingt somit eine perkutane Implantation ohne größeren chirurgischen Eingriff.

## Patentansprüche

1. Vorrichtung mit einer in das Unterhaut-Fettgewebe eines Patienten implantierbaren Infusionskammer, die ein mittels einer Injektionsnadel durchstechbares Punktionsfenster (14, 24, 38, 38') aus unter Vorspannung stehendem und die Einstichstelle selbsttätig wieder schließendem Material besitzt, und mit einem sich von der Infusionskammer forterstreckenden und in ein Blutgefäß, etwa eine Arterie oder eine andere Körperhöhle, einführbaren Katheter,
gekennzeichnet durch die Ausbildung als perkutan durch eine Hautpunktionsöffnung implantierbares System, dessen Infusionskammer (10, 20,30, 30') aus einem langgestreckten formfesten Hohlkörper besteht.

2. Vorrichtung nach Anspruch 1, dadurch gekennzeichnet,
daß es sich bei dem Punktionsfenster um einen die Infusionskammer nach außen abschließenden Stopfen (14, 24, 38, 38') aus Silikon oder einem ähnlichen Material handelt, der unter Vorspannung in einem Teilbereich der Infusionskammer aufgenommen ist.

3. Vorrichtung nach Anspruch 1 oder 2, dadurch gekennzeichnet, daß das Punktionsfenster im Bereich einer die Kammerwand durchbrechenden Ausnehmung (25, 32) angeordnet ist.

4. Vorrichtung nach einem der Ansprüche 1 bis 3, dadurch gekennzeichnet, daß in die Außenzone des das Punktionsfenster bildenden Materials ein das Ausweichen des unter Vorspannung stehenden Fenstermaterials verhinderndes Gitter (43) aus festem Fadenmaterial integriert ist.

5. Vorrichtung nach einem der Ansprüche 1 bis 4, dadurch gekennzeichnet, daß sich um das Punktionsfenster ein über dieses kammeraußenseitig vorstehender Randwulst (45, 45') herumerstreckt.

6. Vorrichtung nach einem der Ansprüche 1 bis 5, dadurch gekennzeichnet, daß die Infusionskammer (30) und der Katheter (35) mittels einer Steckverbindung trennbar miteinander verbunden sind.

7. Vorrichtung nach einem der Ansprüche 1 bis 6, dadurch gekennzeichnet, daß der Querschnitt der Infusionskammer (30') annähernd rechteckförmig ausgebildet ist.

8. Vorrichtung nach einem der Ansprüche 1 bis 7, dadurch gekennzeichnet, daß die Infusionskammer mit sich außenseitig von der Kammerwand forterstreckenden federnden Verankerungsmitteln (26, 27; 44, 44') versehen ist und daß letztere auf der - im implantierten Zustand - Unterseite und gegebenenfalls an den Stirnwänden der Infusionskammer angeordnet sind.

9. Vorrichtung nach Anspruch 8, dadurch gekennzeichnet, daß es sich bei den Verankerungsmitteln um außenseitig von der Kammerwand der Infusionskammer vorstehende Schlingen (26, 44) bzw. Ankerstifte (27) handelt.

10. Vorrichtung nach einem der Ansprüche 1 bis 9, dadurch gekennzeichnet, daß die Infusionskammer (10, 20, 30, 30') mit einem sich in Einführrichtung erstreckenden und zur Infusionskammer hin erweiternden Einführkonus (11, 21, 33) versehen ist.

11. Vorrichtung nach einem der Ansprüche 1 bis 10, dadurch gekennzeichnet, daß die Infusionskammer (10, 20, 30, 30') in einer - nach der Implantation - entgegen der Einführrichtung abziehbaren Einführhülse (28, 52) aufgenommen ist.

## Claims

1. Device with an infusion chamber which is implantable into the subcutaneous fatty tissue of a patient and comprises a puncture window (14, 24, 38, 38'), which is under initial tension, is puncturable by means of a hypodermic needle and which is of a material automatically closing again at the puncture place, and with a catheter which extends forth from the infusion chamber and is introducible into a blood vessel, possibly an artery or another body cavity, characterised by the construction as a system, which is percutaneously implantable through a main puncture opening and the infusion chamber (10, 20, 30, 30') of which consists of an elonate hollow body of firm shape.

2. Device according to claim 1, characterised thereby, that a plug (14, 24, 38, 38') which closes off the infusion chamber outwardly and is of silicone or a similar material, is concerned in the case of the puncture window, which plug is received with prestressing in a part region of the infusion chamber.

3. Device according to claim 1 or 2, characterised thereby, that the puncture window is arranged in the region of a recess (25, 32) penetrating the chamber wall.

4. Device according to one of the claims 1 to 3, characterised thereby, that a grid (43), which is of firm thread material and prevents the deviation of the window material standing under initial tension, is integrated into the material forming the outer zone of the puncture window.

5. Device according to one of the claims 1 to 4, characterised thereby, that a rim bead (45, 45') extends around the puncture window and protrudes beyond this at the outward side of the chamber.

6. Device according to one of the claims 1 to 5, characterised thereby, that the infusion chamber (30) and the catheter (35) are separably connected together by means of a plug connection.

7. Device according to one of the claims 1 to 6, characterised thereby, that the cross-section of the infusion chamber (30') is shaped to be approximately rectangular.

8. Device according to one of the claims 1 to 7, characterised thereby, that the infusion chamber is provided with resilient anchoring means (26, 27; 44, 44'), which extend forth outwardly of the chamber wall and are arranged on that side of the infusion chamber, which is the underside in the implanted state, or in a given case at the end walls of the infusion chamber.

9. Device according to claim 8, characterised thereby, that loops (26, 44) or anchor pins (27), which project outwardly from the chamber wall of the infusion chamber, are concerned in the case of the anchoring means.

10. Device according to one of the claims 1 to 9, characterised thereby, that the infusion chamber (10, 20, 30, 30') is provided with an introductory cone (11, 21, 31), which extends in the direction of introduction and enlarges towards the infusion chamber.

11. Device according to one of the claims 1 to 10, characterised thereby, that the infusion chamber (10, 20, 30, 30') is received in an introductory sleeve (28, 52), which after the implantation can be drawn off against the direction of introduction.

## Revendications

1. Dispositif comprenant une chambre de perfusion pouvant être implantée au moyen d'un cathéter dirigeable en avant dans une artère ou autre conducteur sanguin creux avec une aiguille d'injection, la chambre de perfusion comprenant une fenêtre (14, 24, 38, 38') de prélèvement formée par un matériau auto-obturant, caractérisé en ce qu'il consiste en un système pouvant être implanté à travers une ouverture de ponction dans la peau, la chambre de perfusion (10, 20, 30, 30') étant constituée par un corps creux longitudinal de forme solide.

2. Dispositif selon la revendication 1, caractérisé en ce que dans la fenêtre de ponction, autour de la chambre de perfusion, est prévue un presse-étoupe (14, 24, 38, 38') en silicone ou autre matériau similaire, qui est inséré dans une partie de la chambre de perfusion.

3. Dispositif selon l'une des revendications 1 ou 2, caractérisé en ce que la fenêtre de ponction est disposée dans une paroi de la chambre par une fenêtre traversante (25, 32).

4. Dispositif selon l'une quelconque des revendications 1 à 3, caractérisé en ce dans la zone extérieure du matériau constituant la fenêtre de ponction est intégrée, au dessous du montage du matériau de la fenêtre, une grille (43) constitué de fils.

5. Dispositif selon l'une quelconque des revendications 1 à 4, caractérisé en ce que la fenêtre de ponction est entourée, du côté extérieur de la chambre par un bourrelet (45, 45').

6. Dispositif selon l'une quelconque des revendications 1 à 5, caractérisé en ce que la chambre de perfusion (30) et le cathéter (35) sont reliés par une liaison amovible.

7. Dispositif selon l'une quelconque des revendications 1 à 6, caractérisé en ce que la section de la chambre de perfusion (30') est sensiblement rectangulaire.

8. Dispositif selon l'une quelconque des revendications 1 à 7, caractérisé en ce que la chambre de perfusion est munie de moyens d'ancrage élastiques (26, 27; 44, 44') sur son côté extérieur de paroi et que ces derniers, en position d'implantation, sont disposés du côté inférieur et le cas échéant sur la face frontale de la chambre de perfusion.

9. Dispositif selon la revendication 8, caractérisé en ce que les moyens d'ancrage sur la paroi de la chambre de perfusion consistent en des écharpes (26, 44) ou des tiges d'ancrage (27).

10. Dispositif selon l'une quelconque des revendications 1 à 9, caractérisé en ce que la chambre de perfusion (10, 20, 30, 30') est munie de moyens de guidage, un cône de guidage (11, 21, 33) étant prévu à l'avant de la chambre de perfusion.

11. Dispositif selon l'une quelconque des revendications 1 à 10, caractérisé en ce que la chambre de perfusion (10, 20, 30, 30') est disposée, après implantation, à l'avant du fourneau extractible (28, 52) du dispositif de guidage.
